# FASCICULE DE BREVET EUROPEEN

(11) **EP 3 551 037 B1**
(45) Date de publication et mention de la délivrance du brevet: **01.06.2022**
(21) Numéro de dépôt: 17797402.9
(22) Date de dépôt: 23.10.2017
(51) Int. Cl.: A61B 3/032, A61B 3/00, A61B 3/04

(54) **APPAREIL ET PROCÉDÉ DE MESURE DE RÉFRACTION OCULAIRE SUBJECTIVE DE HAUTE RÉSOLUTION EN PUISSANCE OPTIQUE SPHÉRIQUE ET/OU CYLINDRIQUE**
VORRICHTUNG UND VERFAHREN ZUR MESSUNG DER SUBJEKTIVEN AUGENREFRAKTION MIT HOCHAUFLÖSENDER SPHÄRISCHER UND/ODER ZYLINDRISCHER OPTISCHER LEISTUNG
APPARATUS AND METHOD FOR MEASURING SUBJECTIVE OCULAR REFRACTION WITH HIGH-RESOLUTION SPHERICAL AND/OR CYLINDRICAL OPTICAL POWER

(30) Priorité: 07.12.2016 FR 1662083
(43) Date de publication de la demande: 16.10.2019
(73) Titulaire: Essilor International, 94220 Charenton-le-Pont (FR)
(72) Inventeur: MARIN, Gildas, 94220 Charenton-Le-Pont (FR); PINAULT, Philippe, 94220 Charenton-Le-Pont (FR)
(74) Mandataire: Jacobacci Coralis Harle
(86) Numéro de dépôt international: PCT/FR2017/052915
(87) Numéro de publication internationale: WO 2018/104600

(56) Documents cités:
- WO-A1-02/09579
- WO-A1-2015/155458
- WO-A1-2016/207684
- US-A- 6 048 064
- US-A1- 2007 052 924
- US-A1- 2007 229 762

## Description

### DOMAINE TECHNIQUE AUQUEL SE RAPPORTE L'INVENTION

La présente invention concerne de manière générale le domaine des appareils et méthodes d'optométrie.

Elle concerne plus particulièrement un appareil et une méthode de mesure de réfraction oculaire subjective et/ou de test d'astigmatisme d'une personne.

Elle concerne en particulier un appareil pour la mesure de réfraction oculaire subjective comprenant une mesure de puissance optique sphérique et/ou cylindrique de grande précision.

Elle concerne aussi un procédé de transformation d'un appareil classique pour la mesure de réfraction oculaire subjective ayant une résolution limitée en puissance optique en un appareil de haute précision.

### ARRIERE-PLAN TECHNOLOGIQUE

Dans le cadre de la mesure de réfraction oculaire subjective d'un sujet, il a déjà été proposé de simuler la compensation visuelle à fournir, par exemple au moyen de lunettes d'essai ou d'un réfracteur comme par exemple un phoroptère.

Les lunettes d'essais peuvent accueillir successivement des verres d'essai ayant des corrections différentes de type sphérique et/ou cylindrique, de manière à déterminer de la correction convenant le mieux au patient.

Cette solution nécessite le stockage séparé des verres d'essai dans des boîtes dédiées. Elle implique en outre des changements de lentilles qui provoquent des transitions de puissance de correction non désirées et discontinues. Les verres d'essai sont en général fournis avec une puissance sphérique et/ou cylindrique variable par pas d'au moins 0,25 dioptrie (ou 0.25 D).

Dans un réfracteur, les verres d'essai sont placés sur plusieurs disques, entraînés en rotation manuellement ou à l'aide d'un mécanisme motorisé. Toutefois, la variation de puissance optique est également discontinue par pas d'au moins 0,25 dioptrie.

La précision de mesure de réfraction oculaire subjective est limitée, d'une part, par la capacité du sujet à détecter une différence entre deux optotypes affichés séquentiellement et, d'autre part, par le pas minimum de variation de puissance optique d'un verre d'essai à un autre.

Dans le présent document, un optotype peut être une lettre, un symbole comme par exemple un anneau de Landolt ou, plus généralement, tout dessin ou signe ayant un contraste et un niveau de détail suffisants pour être vu nettement par le sujet. Les optotypes sont de préférence conçus pour présenter un niveau de résolution de détail proche de la perception minimum des détails par l'œil humain.

Le document US 2007/052924 A1 concerne un optomètre pour mesurer la puissance réfractive subjective de l'œil d'un sujet. Le document US 2007/0229762 A1 décrit un appareil de mesure de puissance réfractive subjective comprenant, d'une part, un phoroptère basé sur deux unités symétriques comprenant chacune une pluralité de lentilles de test et, d'autre part, un système d'affichage. Le document US 6,048,064 décrit un appareil de mesure subjective de puissance réfractive comprenant deux unités de lentilles de test, chaque unité comportant une pluralité de lentilles de test et un graphique de test visuel.

Il existe des appareils permettant une mesure de réfraction oculaire subjective utilisant une variation continue de réfraction. Cependant, ces appareils sont généralement complexes et très spécifiques.

Le document de brevet EP 1250883_B1 décrit un appareil d'optométrie comprenant une source de lumière, un objet cible, un système optique à lentille sphérique et/ou cylindrique et un réseau de diffraction formant, pour un œil d'un observateur, différentes images de la cible qui apparaissent dispersées dans un plan transverse à un axe optique et qui sont affichées simultanément à différentes distances virtuelles suivant l'axe optique. Néanmoins, ce système est très complexe et présente des inconvénients de chromatisme et de faible efficacité dus à la diffraction sur le réseau de diffraction.

D'autre part, il existe des méthodes numériques de traitement d'image qui permettent de générer des images pré-corrigées d'optotypes sans système optique conventionnel. Toutefois, ces méthodes numériques sont actuellement peu précises.

Le document WO 2016/507684 A1 décrit une échelle de mesure optométrique et un procédé pour déterminer une valeur de réfraction visuelle d'un individu, l'échelle de mesure optométrique comprenant une pluralité d'optotypes associés à une pluralité de corrections de réfraction visuelle, chaque optotype traité résultant de l'application à un optotype source d'un traitement d'image déterminé associé à une correction de réfraction visuelle définie.

### OBJET DE L'INVENTION

Afin de remédier à l'inconvénient précité de l'état de la technique, la présente invention propose un appareil de mesure de réfraction oculaire subjective comprenant un dispositif de visualisation comprenant un écran configuré pour afficher au moins un optotype, un système optique réfractif disposé entre un œil d'un observateur et le dispositif de visualisation, le système optique réfractif ayant une puissance optique sphérique et/ou cylindrique variable suivant un pas minimum déterminé.

Selon l'invention, le dispositif de visualisation comporte en outre des moyens supplémentaires de variation de puissance optique adaptés pour générer une variation de puissance optique sphérique et/ou cylindrique de manière à ce que le dispositif de visualisation et le système optique réfractif forment une première image de l'optotype avec une première puissance optique totale et, respectivement, une deuxième image de l'optotype avec une deuxième puissance optique totale, la variation de puissance optique entre la première puissance optique totale et la deuxième puissance optique totale étant de valeur non nulle et inférieure en valeur absolue au pas minimum déterminé.

Ainsi, l'appareil permet d'obtenir une mesure de réfraction oculaire subjective avec un pas très fin ou même continu.

Selon un aspect particulier et avantageux de l'invention, le dispositif de visualisation comporte un écran électronique qui comprend un réseau de pixels à deux dimensions, un réseau de microlentilles ou de micro-ouvertures à deux dimensions disposé entre l'écran électronique et le système optique réfractif et les moyens supplémentaires de variation de puissance optique comprennent un système de commande d'affichage sur l'écran électronique, le système de commande étant configuré pour activer une première pluralité de pixels sélectionnés parmi le réseau de pixels pour générer la première image de l'optotype et, respectivement, pour activer une deuxième pluralité de pixels sélectionnés parmi le réseau de pixels pour générer la deuxième image de l'optotype.

Selon un autre aspect particulier et avantageux de l'invention, le dispositif de visualisation comporte un écran électronique qui comprend un réseau de pixels à deux dimensions et dans lequel les moyens supplémentaires de variation de puissance optique comprennent un système de traitement d'image, le système de traitement d'image étant configuré pour appliquer une première précorrection pour générer la première image de l'optotype et pour appliquer une deuxième précorrection pour générer la deuxième image de l'optotype.

Selon un mode de réalisation particulier, les moyens supplémentaires de variation de puissance optique comprennent un composant optique réfractif complémentaire ayant une puissance optique variable spatialement. Dans un exemple, les moyens supplémentaires de variation de puissance optique comprennent un composant optique constitué d'une pluralité de lentilles de focales légèrement différentes les unes des autres. Dans un autre exemple, le composant optique comporte des aberrations d'ordre supérieur à 2 de façon a générer une variation spatiale de puissance optique. Dans un exemple particulier et avantageux, le composant optique réfractif complémentaire comprend une lentille d'Alvarez, une lentille fluidique, une lentille de Fresnel active à base de cristaux liquides ou un modulateur spatial de lumière à base de cristaux liquides.

Selon un autre aspect particulier et avantageux, les moyens supplémentaires de variation de puissance optique comprennent un autre système optique complémentaire constitué d'une première lame et d'une deuxième lame, la première lame ayant une première face plane et une deuxième face ayant un profil de forme polynomiale cubique à deux dimensions et la deuxième lame ayant une deuxième face plane et une première face ayant un profil de forme polynomiale cubique à deux dimensions, la première face de la deuxième lame étant de profil inverse de la deuxième face de la première lame, la deuxième face de la première lame étant disposée en vis à vis la première face de la deuxième lame.

De façon particulièrement avantageuse, l'appareil comporte des moyens opto-mécaniques de déplacement en rotation et/ou en translation du composant optique réfractif complémentaire ou du système optique complémentaire.

Selon un mode de réalisation préféré, la variation de puissance optique comprend une variation de puissance sphérique et/ou une variation de puissance cylindrique et/ou une variation d'orientation d'axe de puissance cylindrique.

Dans un mode de réalisation particulier, la première image de l'optotype et, respectivement, la deuxième image de l'optotype sont générées simultanément suivant une première direction de visée oculaire et, respectivement, suivant une deuxième direction de visée oculaire.

Avantageusement, la première image de l'optotype et la deuxième image de l'optotype sont générées séquentiellement à des instants différents.

Selon un aspect particulier, les moyens supplémentaires de variation de puissance optique sont adaptés pour générer une variation continue de puissance optique dans une gamme limitée de variation de puissance optique de 0.125 dioptrie, 0.25 dioptrie ou 0.5 dioptrie.

De façon avantageuse, les moyens supplémentaires de variation de puissance optique sont adaptés pour générer une variation de puissance optique ayant un pas inférieur ou égal à la moitié du pas minimum déterminé du système optique réfractif.

De façon avantageuse, l'appareil comporte un support soutenant le dispositif de visualisation et le système optique réfractif, le support étant adapté pour être monté sur la tête de l'observateur.

Selon un aspect particulier, l'appareil comporte en outre un système de suivi des mouvements de tête adapté pour déterminer au moins une direction de visée oculaire de l'œil de l'observateur.

L'invention concerne aussi un procédé de transformation d'un appareil de mesure de réfraction oculaire subjective comprenant un dispositif de visualisation comprenant un écran et un système optique réfractif disposé entre un œil d'un observateur et le dispositif de visualisation, le système optique réfractif ayant une puissance optique sphérique et/ou cylindrique variable suivant un pas minimum déterminé, en un appareil de mesure de réfraction oculaire subjective de plus haute résolution en puissance optique sphérique et/ou cylindrique, le procédé de transformation comprenant les étapes suivantes :
- fourniture d'un autre dispositif de visualisation comportant un autre écran configuré pour afficher au moins un optotype et des moyens supplémentaires de variation de puissance optique sphérique et/ou cylindrique adaptés pour générer une variation de puissance optique de manière à ce que le dispositif de visualisation et le système optique réfractif forment une première image de l'optotype avec une première puissance optique totale et, respectivement, une deuxième image de l'optotype avec une deuxième puissance optique totale, la variation de puissance optique entre la première puissance optique totale et la deuxième puissance optique totale étant de valeur non nulle et inférieure en valeur absolue au pas minimum déterminé.

L'invention concerne aussi un procédé de mesure de réfraction oculaire subjective comprenant les étapes suivantes :
- disposer un système optique réfractif entre au moins un œil d'un observateur et un dispositif de visualisation, le système optique réfractif ayant une puissance optique variable selon un pas minimum déterminé, et le dispositif de visualisation comportant en outre des moyens supplémentaires de variation de puissance optique sphérique et/ou cylindrique adaptés pour générer une variation de puissance optique supplémentaire de valeur non nulle et inférieure en valeur absolue au pas minimum déterminé,
- afficher au moins un optotype sur un écran du dispositif de visualisation de manière à ce que le dispositif de visualisation et le système optique réfractif forment une première image de l'optotype,
- faire varier la puissance optique du système optique réfractif suivant le pas minimum déterminé pour déterminer une correction adaptée pour l'œil de l'observateur;
- faire varier la puissance optique des moyens supplémentaires de variation de puissance optique de manière à ce que le dispositif de visualisation et le système optique réfractif forment une deuxième image de l'optotype, pour déterminer une correction de plus haute résolution adaptée pour l'œil de l'observateur.

### DESCRIPTION DETAILLEE D'UN EXEMPLE DE REALISATION

La description qui va suivre en regard des dessins annexés, donnés à titre d'exemples non limitatifs, fera bien comprendre en quoi consiste l'invention et comment elle peut être réalisée.

Sur les dessins annexés :
- la figure 1 représente schématiquement un appareil de mesure de réfraction oculaire subjective et/ou de test d'astigmatisme d'une personne ;
- la figure 2 représente schématiquement une vue en coupe d'un système d'affichage d'optotypes pour un appareil de mesure de réfraction oculaire subjective selon un premier mode de réalisation ;
- la figure 3 représente schématiquement une vue en coupe d'un autre système d'affichage d'optotypes pour un appareil de mesure de réfraction oculaire subjective selon une variante du premier mode de réalisation ;
- les figures 4 à 7 représentent différents exemples d'affichage de tableaux d'optotypes ;
- la figure 8 représente schématiquement un appareil de mesure de réfraction oculaire subjective selon un deuxième mode de réalisation ;
- la figure 9 représente schématiquement une cartographie de lentille de puissance optique variable spatialement.

### Dispositif

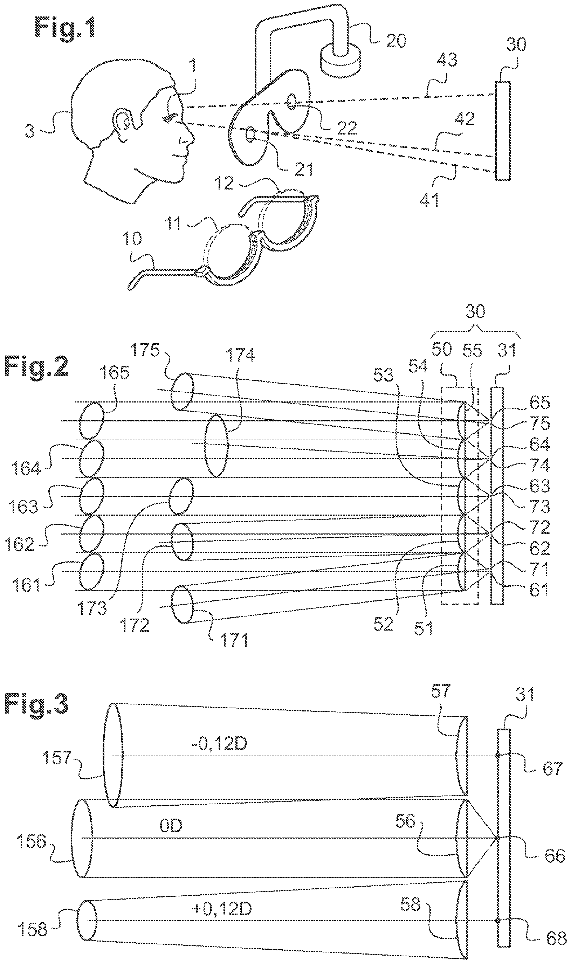

Sur la figure 1, on a représenté un appareil de mesure de réfraction oculaire subjective et/ou de test d'astigmatisme.

L'appareil de la figure 1 comporte une lunette d'essai 10 ou un réfracteur 20 comme par exemple un phoroptère. L'appareil comporte aussi un système d'affichage d'optotypes 30.

Par exemple, la lunette d'essai 10 est arrangée pour recevoir un premier verre d'essai 11 et/ou un deuxième verre d'essai 12. Lorsque le sujet 3 porte la lunette d'essai 10, le premier verre d'essai 11 est disposé devant l'œil 1 droit du sujet 3 et, respectivement, le deuxième verre d'essai 12 est disposé devant l'œil gauche du sujet 3. Le verre d'essai comporte en général une lentille. Chaque lentille présente une puissance optique de type sphérique et/ou cylindrique. L'opticien sélectionne la puissance optique du verre d'essai 11, 12 à partir d'un ensemble de verres d'essai ayant chacun une puissance optique prédéterminée, variant d'un verre à un autre verre par pas d'au moins 0,25 dioptrie. La puissance sphérique et/ou cylindrique du verre d'essai est ainsi variable par pas d'au moins 0,25 dioptrie. Dans le cas d'un verre d'essai cylindrique, différentes orientations de l'axe du cylindre du verre d'essai peuvent être testées pour déterminer l'axe d'astigmatisme de l'œil testé, par exemple suivant des orientations prédéterminées égales à 0 degré, +/- 45 degrés,+/- 90 degrés.

On utilise classiquement un verre d'essai pouvant se décomposer mathématiquement en une lentille sphérique et deux lentilles cylindriques, dites cylindres croisés de Jackson, pour déterminer la correction en sphère, cylindre et axe. On note :
- Sphor la puissance optique sphérique de la lentille sphérique,
- J0phor la puissance optique de la lentille cylindrique à 0 degrés et
- J45phor la puissance optique de la lentille cylindrique à 45 degrés.

De manière équivalente, le réfracteur 20 est configuré pour présenter un verre d'essai 21 devant l'œil 1 droit du sujet 3 et/ou un autre verre d'essai 22 devant l'œil gauche du sujet 3. Différents verres d'essai de puissance optique variable par pas d'au moins 0,25 dioptrie sont intégrés dans le réfracteur. Le réfracteur comporte un mécanisme actionné manuellement ou motorisé pour changer la puissance optique du verre d'essai 21, respectivement 22 disposé devant l'œil 1 droit, respectivement gauche, du sujet 3. La variation de puissance optique du verre d'essai 21, 22 est également discontinue par pas d'au moins 0,25 dioptrie.

Le système d'affichage d'optotypes 30 est configuré pour afficher au moins un premier optotype et un deuxième optotype. Selon les modes de réalisation décrits en détail ci-dessous, le système d'affichage d'optotypes 30 peut comporter un écran passif, par exemple un support imprimé, ou un écran actif comme par exemple un écran optoélectronique. Selon certains modes de réalisation, le premier optotype et le deuxième optotype sont affichés simultanément et visibles suivant des directions de visée oculaire séparées angulairement. Selon d'autres modes de réalisation, le premier optotype et le deuxième optotype sont affichés séquentiellement, de manière à afficher à un instant seulement le premier optotype et à un autre instant seulement le deuxième optotype.

Pendant la mesure de réfraction oculaire subjective, l'œil 1 du sujet 3 visualise à travers le verre d'essai 21 le premier optotype affiché sur le système d'affichage d'optotypes 30, par exemple dans une direction de visée oculaire 41. Sans bouger la tête, l'œil 1 du sujet 3 peut aussi visualiser à travers le verre d'essai 21 le deuxième optotype affiché sur le système d'affichage d'optotypes 30, par exemple dans une deuxième direction de visée oculaire 42.

Selon la présente divulgation, le système d'affichage d'optotypes 30 comporte des moyens supplémentaires de variation de puissance optique adaptés pour générer une variation supplémentaire de puissance optique sphérique et/ou cylindrique inférieure en valeur absolue au pas minimum de variation de puissance optique du verre d'essai. Plus précisément, cette variation supplémentaire de puissance optique sphérique et/ou cylindrique peut être continue ou incrémentale par pas de 0.125 dioptrie. Ainsi, on obtient une mesure de réfraction subjective plus précise qu'avec seulement le phoroptère ou la lunette d'essai.

Nous allons maintenant détailler la structure et le fonctionnement d'un appareil de mesure de réfraction oculaire subjective selon un premier mode de réalisation illustré sur la figure 2.

Dans ce premier mode de réalisation, on utilise une lunette d'essai 10 ou un réfracteur 20 conventionnel et un système d'affichage d'optotypes 30 de type light-field display (LFD ou en français écran d'affichage lumineux). Plus précisément, le système d'affichage d'optotypes 30 comporte un écran de visualisation 31 optoélectronique ayant une matrice de pixels à une dimension ou, de préférence, à deux dimensions. Dans l'exemple de la figure 2, le système d'affichage d'optotypes 30 comporte un réseau de microlentilles 50. Le réseau de microlentilles 50 comporte une pluralité de lentilles 51, 52, 53, 54, 55 disposées en pavage régulier dans un même plan, parallèle au plan de l'écran de visualisation 31. Chaque microlentille 51, respectivement 52, 53, 54, 55 a un point focal situé sur un pixel 61, respectivement 62, 63, 64, 65 de l'écran 31.

On active sélectivement les pixels 61, 62, 63, 64 et/ou 65 pour afficher un point d'un premier optotype. La microlentille 51, respectivement 52, 53, 54, 55 forme un faisceau collimaté 161, respectivement 162, 163, 164, 165 à partir du point source constitué par le pixel 61, respectivement 62, 63, 64, 65. Les faisceaux collimatés 161, 162, 163, 164, 165 sont ici tous parallèles entre eux. On forme ainsi une image du point du premier optotype en activant sélectivement un premier ensemble de pixels 61, 62, 63, 64 et/ou 65. Par rapport à l'œil 1 de l'observateur visualisant les faisceaux 161, 162, 163, 164 et/ou 165 à travers le verre d'essai 21, l'image du point du premier optotype est située à une première distance apparente, ici à l'infini, sur un premier axe de visée oculaire 41.

Simultanément ou séquentiellement, on active sélectivement d'autres pixels 71, 72, 73, 74 et/ou 75 pour afficher un point d'un deuxième optotype. Ces autres pixels 71, 72, 73, 74 et/ou 75 sont en général décalés latéralement par rapport aux pixels 61, 62, 63, 64, 65 situés sur les points focaux du réseau de microlentilles. Ainsi, la microlentille 51, respectivement 52, 53, 54, 55 forme un autre faisceau 171, respectivement 172, 173, 174, 175 à partir du point source constitué par le pixel 71, respectivement 72, 73, 74, 75. L'inclinaison du faisceau 171 est fonction du décalage latéral entre le pixel 71 et le pixel 61 situé au point focal de la microlentille 51. De manière analogue, l'inclinaison du faisceau 172, respectivement 173, 174, 175 est fonction du décalage latéral entre le pixel 72, respectivement 73, 74, 75 et le pixel 62, respectivement 63, 64, 65 situé au point focal de la microlentille 52, respectivement 53, 54, 55. L'ensemble des faisceaux 171, 172, 173, 174, 175 génère ainsi une image du point du deuxième optotype. On forme ainsi l'image du point du deuxième optotype en activant sélectivement un deuxième ensemble de pixels 71, 72, 73, 74 et/ou 75. Par rapport à l'œil 1 de l'observateur visualisant les faisceaux 171, 172, 173, 174 et/ou 175 à travers le verre d'essai 21, l'image du point du deuxième optotype est située à une autre distance apparente que l'image du premier optotype. Pour des facilités de représentation, on a affiché les pixels 61, 62, 63, 64, 65 et les pixels 71,72, 73, 74,75 sur une zone de l'écran 31. En pratique, pour obtenir une séparation spatiale, ces pixels 61, 62, 63, 64, 65 d'une part et 71, 72, 73, 74, 75 d'autre part sont affichés sur des zones séparées de l'écran 31.

De manière analogue, on peut activer sélectivement d'autres pixels pour afficher d'autres points du premier et second optotype simultanément, afin d'afficher l'ensemble des points des premier et second optotypes.

Néanmoins, les optotypes affichés simultanément sont configurés pour que leurs images respectives soient séparées spatialement vues par l'œil 1 de l'observateur à travers le verre d'essai 21.

Ainsi, l'observateur peut visualiser en même temps plusieurs optotypes et par comparaison entre les images du premier optotype, du deuxième optotype et du troisième optotype déterminer aisément l'image qui apparaît la plus nette.

Autrement dit, en déterminant la position des pixels actifs par rapport aux centres des lentilles, il est alors possible de simuler différentes surfaces d'onde, qui peuvent être sphérique ou cylindrique. Dans ce dernier cas, l'espacement entre les pixels n'est pas le même suivant une direction horizontale ou verticale.

De manière générale, lorsque l'écartement entre les pixels actifs 61, 62 est égal à l'écartement entre microlentilles 51,52, alors les faisceaux lumineux 161, 162 issus de ces pixels 61, 62 sont parallèles entre eux et vus comme un point à l'infini. Lorsque l'écartement entre les pixels actifs 73, 74 est supérieur à l'écartement entre microlentilles 53, 54 les faisceaux 173, 174 sont vus comme des faisceaux convergents, simulant ainsi une puissance positive. Lorsque l'écartement entre les pixels actifs 74, 75 est inférieur à l'écartement entre microlentilles 54, 55 les faisceaux 174, 175 sont vus comme des faisceaux divergents, simulant ainsi une puissance négative.

La différence de distance apparente entre l'image du premier optotype et l'image du deuxième optotype par rapport à l'œil 1 de l'observateur est équivalente à une différence de puissance optique. Le système d'affichage d'optotypes 30 est configuré de manière à ce que cette différence de puissance optique soit inférieure au pas minimum de différence de puissance entre deux verres d'essais disponibles. Par exemple, le pas minimum étant égal à 0.25 dioptrie de puissance, le système d'affichage d'optotypes est configuré pour générer une différence de puissance optique entre l'image du premier optotype et l'image du deuxième optotype équivalente à 0.20 dioptrie, ou 0.125 dioptrie, ou 0.10 dioptrie ou 0.05 dioptrie.

On note Sdisplay, J0display et J45display les puissances optiques supplémentaires générées par le système de visualisation 30. Plus précisément, on note Sdisplay1, J0display1 et J45display1 les puissances optiques supplémentaires générées par le dispositif de visualisation correspondant au premier optotype. De même, on note Sdisplay2, J0display2 et J45display2 les puissances optiques supplémentaires générées par le dispositif de visualisation correspondant au deuxième optotype. La différence entre Sdisplay1 et Sdisplay2 est inférieure au pas minimum de variation de puissance sphérique du verre d'essai. De même, la différence entre J0display1 et J0display2 (respectivement entre J45display1 et J45display2) est inférieure au pas minimum de variation de puissance cylindrique des cylindres croisés de Jackson du verre d'essai.

L'observateur 3 observe l'image du premier optotype sur le système de visualisation 30 à travers le verre d'essai, donc à travers un système optique ayant une puissance optique totale égale à la somme de la puissance optique du verre d'essai et de la puissance optique supplémentaire du système de visualisation 30. L'observateur 3 perçoit ainsi le premier optotype avec une puissance optique totale S-1, J0-1, J45-1 égales respectivement à S-1=Sphor+Sdisplay1, J0-1=J0phor+J0display1 et J45-1=J45phor+J45display1. De manière analogue, l'observateur 3 perçoit le deuxième optotype avec une puissance optique totale S-2, J0-2, J45-2 égales respectivement à S-2=Sphor+ Sdisplay2, J0-2=J0phor+J0display2 et J45-2=J45phor+J45display2. La variation de puissance optique entre (S-1, J0-1, J45-1) et (S-2, J0-2, J45-2) est inférieure au pas de variation minium du verre d'essai.

En choisissant un écran 31 à matrice de pixels de très haute résolution, il est ainsi possible de générer une différence de puissance optique variant avec un pas extrêmement fin, de manière quasi-continue. Ainsi, il possible d'obtenir une mesure de réfraction plus précise qu'avec les verres d'essai disponibles.

L'amplitude de la variation supplémentaire de puissance optique ainsi accessible est limitée à une gamme réduite, par exemple de -0.5 à + 0.5 dioptrie mais avec une précision (ou un pas) amélioré par rapport à celui des verres d'essai 21, 22. Moyennant une adaptation du système d'affichage 30, un professionnel peut utiliser un phoroptère ou une lunette d'essai conventionnelle pour réaliser une mesure de réfraction plus précise, sans avoir besoin d'utiliser un équipement spécifique et complexe.

Ainsi, il suffit de remplacer l'écran conventionnel d'un phoroptère conventionnel par un nouveau système de visualisation comprenant des moyens supplémentaires de variation de puissance optique de pas fin pour améliorer considérablement la précision de mesure de réfraction.

La figure 3 représente schématiquement une vue en coupe d'un autre système d'affichage d'optotypes pour un appareil de mesure de réfraction oculaire subjective selon une variante du premier mode de réalisation.

Le système d'affichage de la figure 3 comporte un écran 31 et un système optique complémentaire disposé entre l'écran 31 et le verre d'essai 21. L'écran 31 est de préférence de type optoélectronique. Dans l'exemple de la figure 3, le système optique complémentaire comporte une première lentille 56, une deuxième lentille 57 et une troisième lentille 58. La première lentille 56 est disposée devant une première zone 66 de l'écran 31. De manière analogue, la deuxième lentille 57 est disposée devant une deuxième zone 67 de l'écran 31. La troisième lentille 58 est disposée devant une troisième zone 68 de l'écran 31. La première lentille 56, la deuxième lentille 57 et la troisième lentille 58 ont chacune une puissance optique sphérique et/ou cylindrique différente de l'une de l'autre.

La première lentille 56 est par exemple arrangée telle que son point focal soit situé sur l'écran 31. La première lentille 56 forme un faisceau 156 collimaté à partir de la source constituée par un premier optotype situé dans la première zone 66 de l'écran. La première lentille 56 génère ainsi l'équivalent d'une puissance optique nulle (0D).

La deuxième lentille 57 est par exemple arrangée telle que son point focal soit situé plus loin que l'écran par rapport à cette deuxième lentille. La deuxième lentille 57 forme un deuxième faisceau 157 divergent à partir de la source constituée par un deuxième optotype situé dans la deuxième zone 67 de l'écran. La deuxième lentille 57 génère ainsi l'équivalent d'une puissance optique complémentaire de -0.12 dioptrie.

La troisième lentille 58 est par exemple arrangée telle que son point focal soit situé entre l'écran 31 la troisième lentille 58. La troisième lentille 58 forme un troisième faisceau 158 convergent à partir de la source constituée par un troisième optotype situé dans la troisième zone 68 de l'écran. La troisième lentille 58 génère ainsi l'équivalent d'une puissance optique complémentaire de +0.12 dioptrie.

Sur la figure 3, la première lentille 56 forme l'image d'un premier optotype affiché dans la première zone 66 de l'écran et simultanément, la deuxième lentille 57 forme l'image d'un deuxième optotype affiché dans la deuxième zone 67 de l'écran. De manière analogue, la troisième lentille 58 forme l'image d'un troisième optotype affiché dans la troisième zone 68 de l'écran 31. L'œil 1 de l'observateur 3 visualise ainsi simultanément à travers un verre d'essai 21 une image du premier optotype, une image du deuxième optotype et une image du troisième optotype. Ces images sont séparées spatialement tout en étant affectées d'une différence de puissance complémentaire s'ajoutant à la puissance optique du verre d'essai 21.

Dans un autre mode de réalisation, on utilise un système d'affichage sous forme de tableau d'optotypes, dans lequel chaque image d'optotype est prétraitée de manière à simuler une puissance sphérique et/ou cylindrique complémentaire. Le prétraitement d'image est par exemple basé sur une déconvolution par une fonction de transfert optique d'une puissance optique complémentaire. Dans un autre exemple, le prétraitement d'image est basé sur un calcul de modification de tracé de rayons induit par une puissance optique complémentaire.

Le tableau peut être à une dimension ou à deux dimensions avec un optotype central non modifié et des optotypes périphériques modifiés pour correspondre à un changement de correction d'un pas donné, par exemple par pas de ¼ de dioptrie ou 1/8 de dioptrie. De façon avantageuse, la correction est symétrique et de signe opposé de part et d'autre de l'optotype central. Le tableau peut comporter un nombre impair de lignes et/ou de colonnes par exemple comprenant 3x3, 5x5 ou 7x7 optotypes.

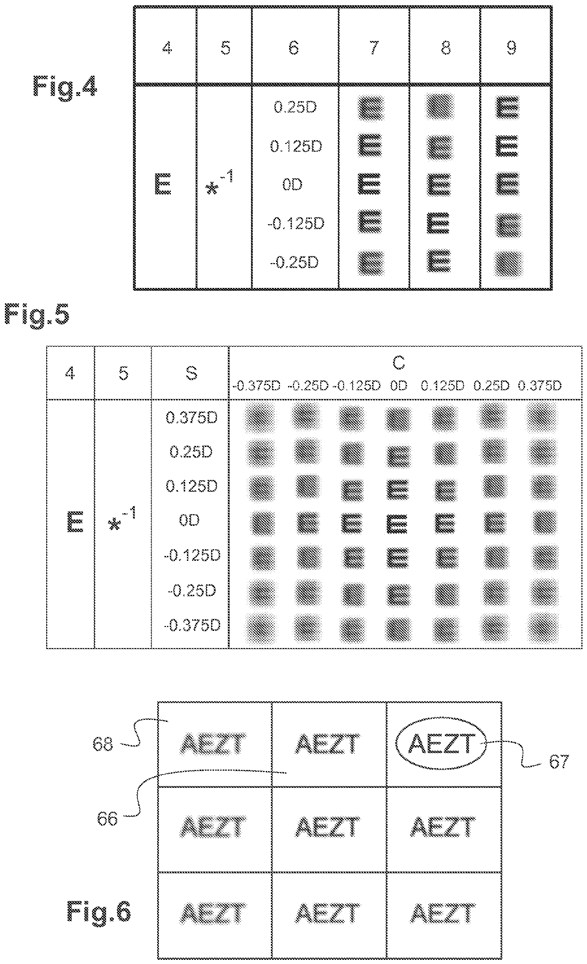

A titre d'exemple, le tableau de la figure 4 représente en colonne 4 un optotype source sans traitement d'image. La colonne 5 représente schématiquement la fonction de traitement d'image utilisée, par exemple une fonction de déconvolution d'une fonction de transfert optique d'une puissance optique complémentaire ou par un calcul de modification de tracé de rayons induit par une puissance optique complémentaire. La colonne 6 représente les différences de puissance optique équivalentes appliquées pour former respectivement un premier optotype (à 0D), un deuxième optotype (+0.125 D), un troisième optotype (-0.125 D), un quatrième optotype (+0.25 D) et un cinquième optotype (-0.25 D). On a ainsi formé une échelle d'optotypes simulant une correction complémentaire allant de -0.25 D à +0.25 D par pas de 0.125 D. La colonne 7 représente comment est vue l'échelle d'optotypes par un observateur n'ayant aucun besoin de correction. Le premier optotype (0 D) au centre de la colonne 7 est vu le plus net, avec un flou croissant symétriquement pour les autres optotypes en fonction d'une différence de puissance croissante. La colonne 8 représente comment est vue l'échelle d'optotypes par un observateur ayant un besoin de correction de -0.125 D. Le troisième optotype (-0.125 D) de la colonne 8 est en effet vu le plus net, avec un flou croissant symétriquement autour de ce troisième optotype (-0.125 D). La colonne 9 représente comment est vue l'échelle d'optotypes par un observateur ayant un besoin de correction de +0.125 D. Le deuxième optotype (+0.125 D) de la colonne 9 est ici vu le plus net, avec un flou croissant symétriquement autour de ce deuxième optotype (+0.125 D). On fait l'hypothèse ici que l'observateur n'accommode pas. Dans le cas contraire, il peut voir net non seulement l'image correspondant à sa réfraction, mais aussi des images correspondant à une puissance négative, tant qu'il a la capacité d'accommoder.

Le tableau la figure 5 utilise deux dimensions pour tester simultanément la modification nécessaire suivant deux paramètres de la correction parmi sphère, cylindre et axe (ou M, J0, J45). Dans ce tableau, les lignes correspondent à une correction sphérique complémentaire allant de -0.375 D à +0.375 D par pas de 0.125 D. Dans ce tableau, les colonnes correspondent à une correction cylindrique complémentaire allant de -0.375 D à +0.375 D par pas de 0.125 D.

Le sujet 3 visualise simultanément tous les optotypes du tableau ce qui permet aisément de comparer la netteté des différentes images d'optotypes avec un pas de différence de puissance optique très fin. Dans cet exemple, l'observateur voit nettement l'optotype correspondant à une puissance optique complémentaire S=0 et C= 0. Dans ce cas, le verre d'essai corrige exactement la réfraction de l'observateur. Dans le cas où l'observateur perçoit un autre optotype le plus nettement parmi le tableau, il peut lire directement la puissance optique complémentaire requise sur l'échelle verticale de correction en sphère et sur l'échelle horizontale de correction en cylindre.

La figure 6 illustre par exemple un écran 31 décomposé en neufs zones, qui permet de faire varier avec un pas fin à la fois la puissance sphérique, la puissance cylindre et l'axe du cylindre. Ici, l'observateur perçoit la zone 67 plus nettement que toutes les autres zones, par exemple 68 et 69 du tableau.

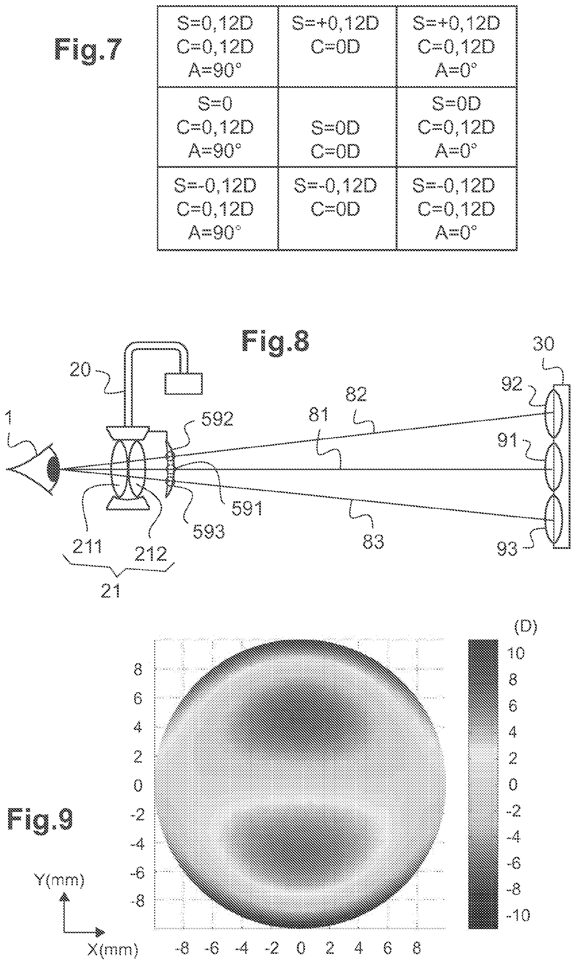

La figure 7 représente les valeurs de sphère, cylindre et axe correspondant à chaque zone de la figure 6. L'opérateur peut ainsi déterminer la correction supplémentaire en sphère, cylindre et axe à ajouter à la correction indiquée par le verre d'essai.

L'écran affiche simultanément plusieurs optotypes (ici une série de lettres : AEZT) dans chaque zone correspondant à une puissance optique sphérique et/ou cylindrique différente.

Préférentiellement, on affiche les mêmes formes d'optotypes dans les différentes zones, afin de faciliter la comparaison visuelle par le sujet.

Le sujet indique la direction vers laquelle le changement est préféré, c'est-à-dire la direction dans laquelle les optotypes apparaissent les plus nets. La correction du phoroptère est modifiée en conséquence, de manière à recentrer l'optotype le plus net dans le tableau et ainsi de suite itérativement jusqu'à obtention d'une correction où l'optotype est vu avec un flou qui va en augmentant de façon parfaitement symétrique de part et d'autre de l'optotype central.

Dans le cas d'un écran combiné à une matrice de lentilles (illustré sur la figure 3), les zones de la figure 4 correspondent idéalement avec les différentes zones 66, 67, 68 définies par la géométrie des lentilles 56, 57, 58.

La figure 8 représente un appareil de mesure de réfraction oculaire subjective selon un troisième mode de réalisation. Dans ce troisième mode de réalisation, on utilise un système d'affichage conventionnel utilisé en combinaison avec le phoroptère ou la lunette d'essai. Le phoroptère 20 comporte par exemple un système optique d'essai comportant par exemple un verre d'essai sphérique 211 et un verre d'essai cylindrique 212 définissant une première correction, relativement grossière suivant le pas de variation de puissance des verres d'essai, en général de ¼ D.

L'appareil comporte en outre une lentille 59 additionnelle présentant une variation spatiale de puissance sur sa surface. Cette lentille 59 additionnelle est par exemple ajoutée à une lunette d'essai ou positionnée entre le phoroptère 20 et l'écran, comme illustré sur la figure 8. La lentille 59 est asphérique et comporte des aberrations optiques géométriques, par exemple de type coma.

La figure 9 illustre schématiquement une représentation graphique de variation spatiale de puissance optique d'une lentille de puissance nulle au centre présentant 0.1 µm de coma (coefficient de Zernike Z 3,3) et d'un diamètre de 20mm. Cette lentille permet de générer une variation spatiale de puissance d'environ 0.1 D/mm le long de l'axe Y de la coma (axe vertical sur la figure 9) donnant un maximum aux bords de +/-1D et une variation similaire en astigmatisme le long de axe X. La rotation de la lentille permet de modifier l'axe de variation de la puissance.

Pour faire varier la puissance, l'appareil comporte avantageusement un dispositif optomécanique de déplacement de cette lentille 59 additionnelle de manière à disposer une zone particulière de la lentille additionnelle dans l'axe visuel du sujet. Le déplacement de la lentille 59 (en direction et en amplitude) permet de déterminer la variation de puissance optique ajoutée.

Dans l'exemple d'implémentation illustré sur la figure 8, toute la surface de la lentille 59 additionnelle est utilisée. Dans ce cas, le système d'affichage présente de préférence au sujet un écran 30 comportant plusieurs zones 91, 92, 93 d'affichage d'optotypes, ces zones 91, 92, 93 étant séparées spatialement. Chaque zone 91, respectivement 92, 93 de l'écran associée à une zone 591, respectivement 592, 593 de la lentille 59 additionnelle. L'axe de visée de l'œil 1 du sujet balaie les différentes zones de la lentille et donc les différentes zones de l'écran afin de tester les différentes valeurs de réfraction.

Ces zones peuvent être affichées séquentiellement, ou de préférence de manière simultanée.

Le sujet en décentrant la lentille devant son œil sélectionne la zone de vision préférée. La connaissance de la correction des verres d'essai 211, 212, de la position exacte de l'œil et de la variation induite par la lentille 59 permet de déduire une nouvelle correction.

Cet appareil permet de faire varier la puissance sphérique et cylindrique de manière continue, ce qui permet ainsi d'augmenter la résolution de l'appareil de mesure de réfraction oculaire subjective utilisé, que ce soit un phoroptère ou une lunette d'essai.

Cet appareil permet d'afficher simultanément des optotypes correspondant à une différence de puissance optique sphérique et/ou cylindrique relativement faible et en tous cas inférieure au pas de variation de puissance des verres d'essai.

Dans une variante, on dispose un autre système optique complémentaire devant l'écran de visualisation. Cet autre système optique est par exemple constitué d'une lentille fluidique. Dans un autre exemple, le système optique complémentaire est constitué une lentille de Fresnel active à base de cristaux liquides ou d'un un dispositif à cristaux liquide formant un pavage de lentille(s) de Fresnel active(s). Une lentille de Fresnel active peut être pilotée électriquement pour générer ou pas une puissance optique complémentaire.

Dans une autre variante, on dispose un autre système optique complémentaire constitué de deux lames, la première lame ayant une première face plane et une deuxième face ayant un profil de forme polynomiale cubique à deux dimensions et la deuxième lame ayant une deuxième face plane et une première face ayant un profil de forme polynomiale cubique à deux dimensions, la première face de la deuxième lame étant de profil inverse de la deuxième face de la première lame et la deuxième face de la première lame étant disposée en vis à vis la première face de la deuxième lame. Par translation le la deuxième lame par rapport à la première lame, on introduit ainsi une variation spatiale de puissance optique par exemple aux ordres supérieurs à l'ordre deux.

Selon un autre mode de réalisation (non représenté sur les figures), le système de visualisation est intégré à un support destiné à être monté directement sur la tête du sujet 3. Par exemple, l'appareil de mesure de réfraction se présente sous la forme d'un casque de réalité virtuelle ou de lunettes à réalité augmentée. Le support est adapté pour recevoir les verres d'essai de puissance optique variable. De plus le support comporte un système de visualisation selon l'un des modes de réalisation décrits ci-dessus. De façon avantageuse, le support permet d'intégrer un système optique supplémentaire disposé entre le verre d'essai et l'écran de visualisation. Ce mode de réalisation a l'avantage d'être très compact. Ce mode de réalisation permet d'utiliser des systèmes optiques complémentaires, à base de lentille fluidique, lentille d'Alvarez, lentille de Fresnel active et/ou lames à profil polynomial de taille réduite. Le mouvement de la tête permet un balayage sur 360°des différentes combinaisons de corrections supplémentaire. Ainsi la navigation dans les couples sphère/cylindre complémentaire se résume à un mouvement de tête sur 2 axes. Le choix se fait ainsi de manière intuitive et naturelle.

De façon particulièrement avantageuse, l'appareil comporte en outre un système de suivi des mouvements de tête (ou tracking de tête) adapté pour déterminer la posture du sujet et/ou pour déterminer une direction de visée oculaire de l'œil de l'observateur.

Ainsi, on détermine la position du regard fixant l'image la plus nette, ce qui permet de déterminer la correction complémentaire en sphère/cylindre par la sélection de l'image correspondante.

### Procédé

La mesure de réfraction oculaire subjective peut être mise en œuvre de différentes manières en utilisant l'un quelconque des appareils décrits ci-dessus.

Dans un exemple de procédé de mesure, on réalise d'abord une première étape mesure de réfraction classique, sans utiliser le système d'affichage et/ou le composant optique additionnel pour faire varier la puissance optique avec un pas plus fin.

Puis, dans une deuxième étape, on affine la mesure avec un pas plus fin. On introduit une variation de puissance de faible amplitude et de pas fin autour de la première mesure de réfraction trouvée à la première étape. Par exemple, sur la figure 6, si le sujet préfère la netteté du test en haut à droite du tableau, on corrige sa réfraction mesurée classiquement et la réfraction complémentaire. Dans cet exemple, la correction sphérique finale est égale à la correction sphérique déterminée à l'étape 1 à laquelle on ajoute une correction sphérique complémentaire de +0,12D.

Dans un autre exemple, on affiche simultanément au moyen du système optique complémentaire et/ou du système d'affichage, différents optotypes correspondant à différentes variations de puissance de réfraction complémentaire sphérico-cylindrique.

De préférence dans ce cas, la valeur centrale de l'optotype affiché correspond à une correction complémentaire sphérique et cylindrique nulle (S=0D, C=0D). Suivant la réponse du porteur sur la netteté préférée dans le tableau affiché, on modifie de manière dynamique la puissance du verre d'essai. Ainsi, dans l'exemple illustré sur la figure 6, on augmente progressivement la puissance de réfraction sphérique et cylindrique. On arrête la mesure de réfraction lorsque la meilleure netteté est obtenue pour l'optotype au centre du tableau.

On peut ainsi déterminer rapidement dans quel sens modifier la réfraction pour atteindre la valeur optimale.

De façon avantageuse, on propose dans la présente divulgation une interface homme-machine (IHM) permettant de faciliter la détermination de la réfraction avec une haute précision et résolution.

Le but est de proposer une méthode de mesure de réfraction subjective facilement utilisable par une personne ne connaissant pas le domaine de la réfraction et sans assistance d'un opérateur qualifié.

La solution générale est d'utiliser l'affichage simultané d'images présentant différentes puissances Sphère/Cylindre/Axe pour aider l'utilisateur à trouver le plus intuitivement possible la réfraction qui lui convient précisément.

Mode de réalisation n°1 :
L'enjeu est de représenter un ensemble de solutions (Rx) définies sur 3 axes (Sph, Cyl, Axe), tout en permettant à l'utilisateur de naviguer simplement et intuitivement dans cet espace afin de trouver le trinôme idéal. Une mise en œuvre sur un écran limite l'espace à 2 dimensions.

Nous proposons différentes mises en œuvre.

Étape 1 : Déceler si le patient est astigmate via un simple test en se plaçant devant un écran avec une mire tel que le cadran de parent.

Si certaines lignes apparaissent grises et d'autres plus noires, (ou certaines lignes floues et d'autres plus nettes), alors le patient est astigmate. L'axe d'astigmatisme sera relevé. Le test est à réaliser séquentiellement sur chacun des deux yeux, en cachant un œil puis l'autre.

Étape 2.1 : Si le patient n'est pas astigmate, la seule valeur à mesurer est la sphère (pas de cylindre). Dans un mode de réalisation, l'écran présente des images déconvoluées correspondant à différentes puissances de sphère. Si l'image est vue nette par le patient, c'est que la sphère correspondant à sa Rx est celle utilisée pour la déconvolution de l'image. Pour une expérience utilisateur simplifiée, le patient navigue avec les flèches gauche/droite du pavé numérique si le test est réalisé sur un ordinateur ou en faisant glisser les images si le périphérique est tactile (téléphone intelligent ou tablette par exemple). Ce test devra être fait indépendamment pour les deux yeux, en cachant un œil puis l'autre.

Etape 2.2 : Si le patient est astigmate, on suppose que le ou les axes d'astigmatisme sont connus), il s'agit de déterminer la valeur de la puissance de sphère et la valeur de la puissance de cylindre. Les différentes valeurs de S et C peuvent être présentées en deux dimensions. Par exemple, on présente des images déconvoluées correspondant à différents couples en sphère et cylindre. Si l'image est vue nette par le patient, c'est que le couple sphère/cylindre correspond à sa mesure de réfraction. Pour une expérience utilisateur simplifiée, le patient navigue avec les flèches gauche/droite du pavé numérique pour la sphère et haut/bas pour naviguer pour le cylindre si le test est réalisé sur un ordinateur ou en faisant glisser les images si le périphérique est tactile (téléphone intelligent ou tablette par exemple). Ce test devra être fait indépendamment pour les deux yeux, en cachant un œil puis l'autre.

De façon particulièrement avantageuse, si l'appareil comporte en outre un système de suivi des mouvements de tête (ou tracking de tête) adapté pour déterminer la posture du sujet et/ou pour déterminer une direction de visée oculaire de l'œil de l'observateur, on détermine la position du regard fixant l'image la plus nette, ce qui permet de déterminer la correction complémentaire en sphère/cylindre par la sélection de l'image correspondante.

## Revendications

1. Appareil de mesure de réfraction oculaire subjective comprenant :
- un dispositif de visualisation comprenant un écran configuré pour afficher au moins un optotype ;
- un système optique réfractif (11, 21) disposé entre un œil d'un observateur et le dispositif de visualisation, le système optique réfractif ayant une puissance optique sphérique et/ou cylindrique variable suivant un pas minimum déterminé;
**caractérisé en ce que** :
- le dispositif de visualisation comporte en outre des moyens supplémentaires de variation de puissance optique adaptés pour générer une variation de puissance optique sphérique et/ou cylindrique de manière à ce que le dispositif de visualisation et le système optique réfractif forment une première image de l'optotype avec une première puissance optique totale et, respectivement, une deuxième image de l'optotype avec une deuxième puissance optique totale, la variation de puissance optique entre la première puissance optique totale et la deuxième puissance optique totale étant de valeur non nulle et inférieure en valeur absolue au pas minimum déterminé.

2. Appareil selon la revendication 1, dans lequel le dispositif de visualisation comporte un écran électronique (31) comprenant un réseau de pixels à deux dimensions, un réseau de microlentilles (50) ou de micro-ouvertures à deux dimensions disposé entre l'écran électronique (31) et le système optique réfractif (11, 21), et dans lequel les moyens supplémentaires de variation de puissance optique comprennent un système de commande d'affichage sur l'écran électronique, le système de commande étant configuré pour activer une première pluralité de pixels (61, 62, 63, 64, 65) sélectionnés parmi le réseau de pixels pour générer la première image de l'optotype et, respectivement, pour activer une deuxième pluralité de pixels (71, 72, 73, 74, 75) sélectionnés parmi le réseau de pixels pour générer la deuxième image de l'optotype.

3. Appareil selon la revendication 1 dans lequel le dispositif de visualisation comporte un écran électronique (31) comprenant un réseau de pixels à deux dimensions et dans lequel les moyens supplémentaires de variation de puissance optique comprennent un système de traitement d'image, le système de traitement d'image étant configuré pour appliquer une première précorrection pour générer la première image de l'optotype et pour appliquer une deuxième précorrection pour générer la deuxième image de l'optotype.

4. Appareil selon l'une des revendications 1 à 3 dans lequel les moyens supplémentaires de variation de puissance optique comprennent un composant optique réfractif complémentaire (59) ayant une puissance optique variable spatialement.

5. Appareil selon la revendication 4 dans lequel le composant optique réfractif complémentaire (59) comprend une lentille d'Alvarez, une lentille fluidique, une lentille de Fresnel active à base de cristaux liquides ou un modulateur spatial de lumière à base de cristaux liquides.

6. Appareil selon l'une des revendications 1 à 5 dans lequel les moyens supplémentaires de variation de puissance optique comprennent un autre système optique complémentaire constitué d'une première lame et d'une deuxième lame, la première lame ayant une première face plane et une deuxième face ayant un profil de forme polynomiale cubique à deux dimensions et la deuxième lame ayant une deuxième face plane et une première face ayant un profil de forme polynomiale cubique à deux dimensions, la première face de la deuxième lame étant de profil inverse de la deuxième face de la première lame, la deuxième face de la première lame étant disposée en vis à vis de la première face de la deuxième lame.

7. Appareil selon l'une des revendications 4 à 6 comportant des moyens opto-mécaniques de déplacement en rotation et/ou en translation du composant optique réfractif complémentaire (59) ou du système optique complémentaire.

8. Appareil selon l'une des revendications 1 à 7, dans lequel la variation de puissance optique comprend une variation de puissance sphérique et/ou une variation de puissance cylindrique et/ou une variation d'orientation d'axe de puissance cylindrique.

9. Appareil selon l'une des revendications 1 à 8, dans lequel le dispositif de visualisation et le système optique réfractif sont adaptés pour générer simultanément la première image de l'optotype suivant une première direction de visée oculaire et, respectivement, la deuxième image de l'optotype suivant une deuxième direction de visée oculaire.

10. Appareil selon l'une des revendications 1 à 8, dans lequel le dispositif de visualisation et le système optique réfractif sont adaptés pour générer séquentiellement la première image de l'optotype et la deuxième image de l'optotype à des instants différents.

11. Appareil selon l'une des revendications 1 à 10 dans lequel les moyens supplémentaires de variation de puissance optique sont adaptés pour générer une variation continue de puissance optique dans une gamme limitée de variation de puissance optique de 0.125 dioptrie, 0.25 dioptrie ou 0.5 dioptrie.

12. Appareil selon l'une des revendications 1 à 10 dans lequel les moyens supplémentaires de variation de puissance optique sont adaptés pour générer une variation de puissance optique ayant un pas inférieur ou égal à la moitié du pas minimum déterminé du système optique réfractif (11, 21).

13. Appareil selon l'une des revendications 1 à 12 comportant en outre un système de suivi des mouvements de tête adapté pour déterminer au moins une direction de visée oculaire de l'œil de l'observateur.

14. Procédé de transformation d'un appareil de mesure de réfraction oculaire subjective comprenant un dispositif de visualisation comprenant un écran et un système optique réfractif disposé entre un œil d'un observateur et le dispositif de visualisation, le système optique réfractif ayant une puissance optique sphérique et/ou cylindrique variable suivant un pas minimum déterminé, en un appareil de mesure de réfraction oculaire subjective de plus haute résolution en puissance optique sphérique et/ou cylindrique, le procédé de transformation comprenant les étapes suivantes :
- fourniture d'un autre dispositif de visualisation comportant un autre écran configuré pour afficher au moins un optotype et des moyens supplémentaires de variation de puissance optique sphérique et/ou cylindrique adaptés pour générer une variation de puissance optique de manière à ce que le dispositif de visualisation et le système optique réfractif forment une première image de l'optotype avec une première puissance optique totale et, respectivement, une deuxième image de l'optotype avec une deuxième puissance optique totale, la variation de puissance optique entre la première puissance optique totale et la deuxième puissance optique totale étant de valeur non nulle et inférieure en valeur absolue au pas minimum déterminé.

15. Procédé de mesure de réfraction oculaire subjective comprenant les étapes suivantes :
- disposer un système optique réfractif (11, 21) entre au moins un œil (1) d'un observateur (3) et un dispositif de visualisation (30, 31), le système optique réfractif (11, 21) ayant une puissance optique variable selon un pas minimum déterminé, et le dispositif de visualisation comportant en outre des moyens supplémentaires de variation de puissance optique sphérique et/ou cylindrique adaptés pour générer une variation de puissance optique supplémentaire de valeur non nulle et inférieure en valeur absolue au pas minimum déterminé,
- afficher au moins un optotype sur un écran du dispositif de visualisation (30, 31) de manière à ce que le dispositif de visualisation et le système optique réfractif forment une première image de l'optotype,
- faire varier la puissance optique du système optique réfractif (11, 21) suivant le pas minimum déterminé pour déterminer une correction adaptée pour l'œil (1) de l'observateur (3);
- faire varier la puissance optique des moyens supplémentaires de variation de puissance optique de manière à ce que le dispositif de visualisation et le système optique réfractif forment une deuxième image de l'optotype, pour déterminer une correction de plus haute résolution adaptée pour l'œil (1) de l'observateur (3).

## Patentansprüche

1. Gerät zur Messung der subjektiven Refraktion des Auges, umfassend:
- eine Anzeigevorrichtung, die einen Bildschirm umfasst, der dazu ausgestaltet ist, mindestens ein Sehzeichen anzuzeigen;
- ein refraktives optisches System (11, 21), das zwischen einem Auge eines Betrachters und der Anzeigevorrichtung angeordnet ist, wobei das refraktive optische System eine optische sphärische und/oder Zylinderstärke hat, die gemäß einer bestimmten Mindestschrittweite variabel ist; **dadurch gekennzeichnet, dass**:
- die Anzeigevorrichtung ferner zusätzliche Mittel zum Ändern der optischen Stärke beinhaltet, die dazu geeignet sind, eine Änderung der optischen sphärischen und/oder Zylinderstärke derart zu erzeugen, dass die Anzeigevorrichtung und das refraktive optische System ein erstes Bild des Sehzeichens mit einer ersten optischen Gesamtstärke beziehungsweise ein zweites Bild des Sehzeichens mit einer zweiten optischen Gesamtstärke bilden, wobei die Änderung der optischen Stärke zwischen der ersten optischen Gesamtstärke und der zweiten optischen Gesamtstärke einen Wert hat, der ungleich null ist und als Absolutwert kleiner als die bestimmte Mindestschrittweite ist.

2. Gerät nach Anspruch 1 bei dem die Anzeigevorrichtung einen elektronischen Bildschirm (31) beinhaltet, der ein zweidimensionales Pixelgitter, ein zweidimensionales Mikrolinsen- oder Mikroöffnungsgitter (50) umfasst, das zwischen dem elektronischen Bildschirm (31) und dem refraktiven optischen System (11, 21) angeordnet ist, und bei dem die zusätzlichen Mittel zum Ändern der optischen Stärke ein System zum Steuern der Anzeige auf dem elektronischen Bildschirm umfassen, wobei das Steuerungssystem dazu ausgestaltet ist, eine erste Mehrzahl von Pixeln (61, 62, 63, 64, 65) zu aktivieren, die aus dem Pixelgitter ausgewählt sind, um das erste Bild des Sehzeichens zu erzeugen, beziehungsweise eine zweite Mehrzahl von Pixeln (71, 72, 73, 74, 75) zu aktivieren, die aus dem Pixelgitter ausgewählt sind, um das zweite Bild des Sehzeichens zu erzeugen.

3. Gerät nach Anspruch 1, bei dem die Anzeigevorrichtung einen elektronischen Bildschirm (31) beinhaltet, der ein zweidimensionales Pixelgitter umfasst, und bei dem die zusätzlichen Mittel zum Ändern der optischen Stärke ein Bildverarbeitungssystem umfassen, wobei das Bildverarbeitungssystem dazu ausgestaltet ist, eine erste Vorkorrektur anzuwenden, um das erste Bild des Sehzeichens zu erzeugen, und eine zweite Vorkorrektur anzuwenden, um das zweite Bild des Sehzeichens zu erzeugen.

4. Gerät nach einem der Ansprüche 1 bis 3, bei dem die zusätzlichen Mittel zum Ändern der optischen Stärke eine ergänzende refraktive optische Komponente (59) umfassen, die eine räumlich variable optische Stärke hat.

5. Gerät nach Anspruch 4, bei dem die ergänzende refraktive optische Komponente (59) eine Alvarez-Linse, eine fluidische Linse, eine aktive Fresnel-Linse auf Basis von Flüssigkristallen oder einen räumlichen Modulator für Licht auf Basis von Flüssigkristallen umfasst.

6. Gerät nach einem der Ansprüche 1 bis 5, bei dem die zusätzlichen Mittel zum Ändern der optischen Stärke ein weiteres ergänzendes optisches System umfassen, das aus einem ersten Plättchen und aus einem zweiten Plättchen besteht, wobei das erste Plättchen eine erste plane Fläche hat und eine zweite plane Fläche, die ein zweidimensionales Profil von kubischer polynomialer Form hat, und das zweite Plättchen eine zweite plane Fläche hat und eine erste Fläche, die ein zweidimensionales Profil von kubischer polynomialer Form hat, wobei die erste Fläche des zweiten Plättchens das inverse Profil der zweiten Fläche des ersten Plättchens hat, wobei die zweite Fläche des ersten Plättchens gegenüber der ersten Fläche des zweiten Plättchens angeordnet ist.

7. Gerät nach einem der Ansprüche 4 bis 6, das optomechanische Mittel zur rotatorischen und/oder translatorischen Verlagerung der ergänzenden refraktiven optischen Komponente (59) oder des ergänzenden optischen Systems beinhaltet.

8. Gerät nach einem der Ansprüche 1 bis 7, bei dem die Änderung der optischen Stärke eine Änderung der sphärischen Stärke und/oder eine Änderung der Zylinderstärke und/oder eine Änderung der Ausrichtung der Achse der Zylinderstärke umfasst.

9. Gerät nach einem der Ansprüche 1 bis 8, bei dem die Anzeigevorrichtung und das refraktive optische System dazu geeignet sind, gleichzeitig das erste Bild des Sehzeichens gemäß einer ersten Blickrichtung beziehungsweise das zweite Bild des Sehzeichens gemäß einer zweiten Blickrichtung zu erzeugen.

10. Gerät nach einem der Ansprüche 1 bis 8, bei dem die Anzeigevorrichtung und das refraktive optische System dazu geeignet sind, das erste Bild des Sehzeichens und das zweite Bild des Sehzeichens sequenziell zu unterschiedlichen Zeitpunkten zu erzeugen.

11. Gerät nach einem der Ansprüche 1 bis 10, bei dem die zusätzlichen Mittel zum Ändern der optischen Stärke dazu geeignet sind, eine stufenlose Änderung der optischen Stärke in einem begrenzen Änderungsbereich der optischen Stärke von 0,125 Dioptrien, 0,25 Dioptrien oder 0,5 Dioptrien zu erzeugen.

12. Gerät nach einem der Ansprüche 1 bis 10, bei dem die zusätzlichen Mittel zum Ändern der optischen Stärke dazu geeignet sind, eine Änderung der optischen Stärke mit einer Schrittweite zu erzeugen, die kleiner oder gleich der Hälfte der bestimmten Mindestschrittweite des refraktiven optischen Systems (11, 21) ist.

13. Gerät nach einem der Ansprüche 1 bis 12, das ferner ein System zur Nachverfolgung der Kopfbewegungen beinhaltet, das dazu geeignet ist, mindestens eine Blickrichtung des Auges des Betrachters zu bestimmen.

14. Verfahren zur Umwandlung eines Geräts zur Messung der subjektiven Refraktion des Auges, das eine Anzeigevorrichtung umfasst, die einen Bildschirm umfasst und ein refraktives optisches System, das zwischen einem Auge eines Betrachters und der Anzeigevorrichtung angeordnet ist, wobei das refraktive optische System eine optische sphärische und/oder Zylinderstärke hat, die gemäß einer bestimmten Mindestschrittweite variabel ist, in ein Gerät zur Messung der subjektiven Refraktion des Auges mit höherer Auflösung hinsichtlich der optischen sphärischen und/oder Zylinderstärke, wobei das Umwandlungsverfahren die folgenden Schritte umfasst:
- Bereitstellen einer anderen Anzeigevorrichtung, die einen anderen Bildschirm beinhaltet, der dazu ausgestaltet ist, mindestens ein Sehzeichens anzuzeigen, und zusätzliche Mittel zum Ändern der optischen sphärischen und/oder Zylinderstärke, die dazu geeignet sind, eine Änderung der optischen Stärke derart zu erzeugen, dass die Anzeigevorrichtung und das refraktive optische System ein erstes Bild des Sehzeichens mit einer ersten optischen Gesamtstärke beziehungsweise ein zweites Bild des Sehzeichens mit einer zweiten optischen Gesamtstärke bilden, wobei die Änderung der optischen Stärke zwischen der ersten optischen Gesamtstärke und der zweiten optischen Gesamtstärke einen Wert hat, der ungleich null ist und als Absolutwert kleiner als die bestimmte Mindestschrittweite ist.

15. Verfahren zur Messung der subjektiven Refraktion des Auges, das die folgenden Schritte umfasst:
- Anordnen eines refraktiven optischen Systems (11, 21) zwischen mindestens einem Auge (1) eines Betrachters (3) und einer Anzeigevorrichtung (30, 31), wobei das refraktive optische System (11, 21) eine gemäß einer bestimmten Mindestschrittweite variable optische Stärke hat und wobei die Anzeigevorrichtung ferner zusätzliche Mittel zum Ändern der optischen sphärischen und/oder Zylinderstärke umfasst, die dazu geeignet sind, eine zusätzliche Änderung der optischen Stärke zu erzeugen, deren Wert ungleich null und als Absolutwert kleiner als die bestimmte Mindestschrittweite ist,
- Anzeigen mindestens eines Sehzeichens auf einem Bildschirm der Anzeigevorrichtung (30, 31) derart, dass die Anzeigevorrichtung und das refraktive optische System ein erstes Bild des Sehzeichens bilden,
- Ändern der optischen Stärke des refraktiven optischen Systems (11, 21) gemäß der bestimmten Mindestschrittweite, um eine für das Auge (1) des Betrachters (3) geeignete Korrektur zu bestimmen;
- Ändern der optischen Stärke der zusätzlichen Mittel zum Ändern der optischen Stärke derart, dass die Anzeigevorrichtung und das refraktive optische System ein zweites Bild des Sehzeichens bilden, um eine für das Auge (1) des Betrachters (3) geeignete Korrektur mit höherer Auflösung zu bestimmen.

## Claims

1. Apparatus for measuring subjective ocular refraction comprising:
- a viewing device comprising a screen configured to display at least one optotype;
- a refractive optical system (11, 21) placed between an eye of an observer and the viewing device, the refractive optical system having a cylindrical and/or spherical optical power that may be varied in steps of a determined minimum step size;
**characterized in that**:
- the viewing device furthermore includes additional means for varying optical power, said means being suitable for generating a variation in cylindrical and/or spherical optical power so that the viewing device and the refractive optical system form a first image of the optotype with a first total optical power and a second image of the optotype with a second total optical power, respectively, the variation in optical power between the first total optical power and the second total optical power being of nonzero value and lower in absolute value than the determined minimum step size.

2. Apparatus according to Claim 1, wherein the viewing device includes an electronic screen (31) comprising a two-dimensional array of pixels and a two-dimensional array of micro-lenses (50) or of micro-apertures that is placed between the electronic screen (31) and the refractive optical system (11, 21), and wherein the additional means for varying optical power comprise a system for controlling the display on the electronic screen, the control system being configured to activate a first plurality of pixels (61, 62, 63, 64, 65) selected from among the array of pixels in order to generate the first image of the optotype and to activate a second plurality of pixels (71, 72, 73, 74, 75) selected from among the array of pixels in order to generate the second image of the optotype, respectively.

3. Apparatus according to Claim 1, wherein the viewing device includes an electronic screen (31) comprising a two-dimensional array of pixels and wherein the additional means for varying optical power comprise an image-processing system, the image-processing system being configured to apply a first pre-correction to generate the first image of the optotype and to apply a second pre-correction to generate the second image of the optotype.

4. Apparatus according to one of Claims 1 to 3, wherein the additional means for varying optical power comprise a complementary refractive optical component (59) having a spatially variable optical power.

5. Apparatus according to Claim 4, wherein the complementary refractive optical component (59) comprises an Alvarez lens, a liquid lens, an active Fresnel lens based on liquid crystals or a spatial modulator of light based on liquid crystals.

6. Apparatus according to one of Claims 1 to 5, wherein the additional means for varying optical power comprise another complementary optical system consisting of a first plate and of a second plate, the first plate having a planar first face and a second face having a two-dimensional profile of cubic polynomial form and the second plate having a planar second face and a first face having a two-dimensional profile of cubic polynomial form, the first face of the second plate being of inverse profile to the second face of the first plate, the second face of the first plate being placed facing the first face of the second plate.

7. Apparatus according to one of Claims 4 to 6, including opto-mechanical means for translating and/or rotating the complementary refractive optical component (59) or the complementary optical system.

8. Apparatus according to one of Claims 1 to 7, wherein the variation in optical power comprises a variation in spherical power and/or a variation in cylindrical power and/or a variation in the orientation of the cylindrical-power axis.

9. Apparatus according to one of Claims 1 to 8, wherein the viewing device and the refractive optical system are suitable for generating, simultaneously, the first image of the optotype in a first direction of ocular sight and the second image of the optotype in a second direction of ocular sight, respectively.

10. Apparatus according to one of Claims 1 to 8, wherein the viewing device and the refractive optical system are suitable for generating, sequentially, the first image of the optotype and the second image of the optotype at different times.

11. Apparatus according to one of Claims 1 to 10, wherein the additional means for varying optical power are suitable for generating a continuous variation in optical power in a limited range of variation in optical power of 0.125 diopters, 0.25 diopters or 0.5 diopters.

12. Apparatus according to one of Claims 1 to 10, wherein the additional means for varying optical power are suitable for generating a variation in optical power having a step size smaller than or equal to half the determined minimum step size of the refractive optical system (11, 21).

13. Apparatus according to one of Claims 1 to 12, furthermore including a system for tracking head movements suitable for determining at least one ocular sight direction of the eye of the observer.

14. Method for converting an apparatus for measuring subjective ocular refraction comprising a viewing device comprising a screen and a refractive optical system placed between an eye of an observer and the viewing device, the refractive optical system having a cylindrical and/or spherical optical power that may be varied in steps of a determined minimum step size, into an apparatus for measuring subjective ocular refraction of higher resolution in cylindrical and/or spherical optical power, the converting method comprising the following steps:
- providing another viewing device including another screen configured to display a least one optotype and additional means for varying cylindrical and/or spherical optical power that are suitable for generating a variation in optical power so that the viewing device and the refractive optical system form a first image of the optotype with a first total optical power and a second image of the optotype with a second total optical power, respectively, the variation in optical power between the first total optical power and the second total optical power being of nonzero value and lower in absolute value than the determined minimum step size.

15. Method for measuring subjective ocular refraction, comprising the following steps:
- placing a refractive optical system (11, 21) between at least one eye (1) of an observer (3) and a viewing device (30, 31), the refractive optical system (11, 21) having an optical power that may be varied in steps of a determined minimum step size, and the viewing device furthermore including additional means for varying cylindrical and/or spherical optical power that are suitable for generating an additional variation in optical power of nonzero value and lower in absolute value than the determined minimum step size,
- displaying at least one optotype on a screen of the viewing device (30, 31) so that the viewing device and the refractive optical system form a first image of the optotype,
- making the optical power of the refractive optical system (11, 21) vary by the determined minimum step size in order to determine a correction suitable for the eye (1) of the observer (3);
- making the optical power of the additional means for varying optical power vary so that the viewing device and the refractive optical system form a second image of the optotype, in order to determine a correction of higher resolution suitable for the eye (1) of the observer (3).
